(19) Europäisches Patentamt
European Patent Office
Office européen des brevets

(11) EP 0 791 349 B1

(12) **FASCICULE DE BREVET EUROPEEN**

(45) Date de publication et mention de la délivrance du brevet:
**20.06.2001 Bulletin 2001/25**

(51) Int Cl.[7]: **A61K 7/00**, A61K 7/06

(21) Numéro de dépôt: **97400388.1**

(22) Date de dépôt: **21.02.1997**

(54) **Composition cosmetique pour mousse capillaire**

Kosmetische Zusammensetzung für Frisierschaum

Cosmetic composition for hair-styling mousse

(84) Etats contractants désignés:
**DE ES FR GB IT**

(30) Priorité: **21.02.1996 FR 9602125**

(43) Date de publication de la demande:
**27.08.1997 Bulletin 1997/35**

(73) Titulaire: **L'OREAL**
**75008 Paris (FR)**

(72) Inventeur: **Duphuis, Christine**
**75018 Paris (FR)**

(74) Mandataire: **Stalla-Bourdillon, Bernard**
**NONY & ASSOCIES,**
**3, rue de Penthièvre**
**75008 Paris (FR)**

(56) Documents cités:
**EP-A- 0 172 713**
**WO-A-91/03521**
**GB-A- 2 098 624**
**US-A- 4 985 487**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 791 349 B1

## Description

La présente invention a pour objet une composition pour la formation d'une mousse capillaire comprenant au moins un polymère cationique et au moins un polymère amphotère, présentant de bonnes propriétés moussantes et conduisant à la formation d'une mousse présentant d'excellentes qualités cosmétiques.

Par propriétés moussantes, on entend selon la présente invention des propriétés permettant d'obtenir une mousse répondant aux exigences des consommateurs quant aux caractéristiques d'expansion, de rigidité et de stabilité. Une telle mousse doit par ailleurs avoir une masse volumique inférieure à 0,4 et de préférence inférieure à 0,25 g/$cm^3$ (les conditions opératoires de ce test seront décrites plus en détail ci-après).

Il a déjà été décrit dans le brevet FR-82.07996 (2.505.348) une composition pour mousse aérosol contenant au moins un polymère cationique et/ou un polymère anionique dans un milieu solvant aqueux, sans adjonction d'un composé tensioactif moussant, le polymère cationique et/ou le polymère anionique ayant des propriétés moussantes permettant d'obtenir, en combinaison avec le milieu solvant utilisé, une mousse éphémère disparaissant rapidement au contact de la chevelure sans laisser de mousse résiduelle.

Ce brevet décrit également une association dans laquelle le polymère anionique et/ou le polymère cationique peut être associé à un polymère amphotère.

Parmi les polymères cationiques connus, certains présentent des propriétés moussantes médiocres telles qu'il s'avère nécessaire de leur associer un polymère amphotère à propriétés moussantes suffisantes pour que l'association en résultant conduise à une mousse répondant aux exigences cosmétiques requises.

Ainsi, dans le brevet FR-82.07996, on préconise, notamment en association avec un polymère cationique, divers polymères amphotères dont notamment le copolymère N-octylacrylamide/acide acrylique/t-butylaminoéthyl méthcrylate ("Amphomer"® de la Société NATIONAL STARCH) qui est connu pour présenter de bonnes propriétés moussantes. Toutefois, ce type de polymère amphotère présente des propriétés cosmétiques non satisfaisantes dans la mesure où il ne confère aux cheveux qu'une douceur moyenne et pose même des problèmes de crissement des cheveux au démêlage (voir en particulier "The Science of Hair Care", Ch. ZVIAK, p.163 : "Amphoteric resins").

Il a maintenant été trouvé de manière inattendue et tout à fait surprenante que l'association d'une sélection de certains polymères cationiques avec une classe bien définie de polymères amphotères qui pris isolément présentent des propriétés moussantes médiocres, permettait d'obtenir un effet de synergie marquée quant aux caractéristiques d'expansion, de rigidité et de stabilité de la mousse obtenue, celle-ci présentant, en outre, une masse volumique avantageusement inférieure à 0,25 g/$cm^3$. Par ailleurs, cette association permet d'obtenir des propriétés cosmétiques avantageuses notamment quant à la douceur et au démêlage des cheveux.

Ainsi, la présente invention a pour objet une composition cosmétique pour mousse capillaire à base d'une association de polymères, caractérisée par le fait qu'elle contient, dans un véhicule cosmétiquement acceptable, un mélange (i) d'au moins un polymère cationique choisi parmi les celluloses cationiques, les éthers de cellulose comportant des groupements ammonium quaternaires, les copolymères de vinyl pyrrolidone et d'acrylate ou méthacrylate de dialkylaminoalkyle quaternisés ou non quaternisés, les copolymères de vinyl pyrrolidone et d'acrylamide ou méthacrylamide de dialkyl-aminoalkyle quaternisés ou non quaternisés et (ii) d'au moins un polymère amphotère choisi parmi les polymères dérivant de la copolymérisation d'un alkylvinyléther et d'anhydride maléique, d'un N-vinyl lactame et d'anhydride maléique, ou d'un alkylvinyléther, d'anhydride maléique et d'un N-vinyl lactame, lesdits polymères étant modifiés par une N,N-dialkylaminoalkylamine ou par un N,N-dialkylaminoalcanol.

Lorsque le polymère cationique des compositions selon l'invention est une cellulose cationique, cette dernière est de préférence choisie parmi les dérivés de cellulose greffés par un monomère hydrosoluble sous forme ammonium quaternaire tels que ceux décrits dans le brevet US-4.131.576 et en particulier les hydroxy-alkylcelluloses telles que les hydroxyméthyl-, hydroxyéthyl- ou hydroxypropyl-celluloses greffées par un sel de méthacryloyl-éthyltriméthyl ammonium, de méthacrylamidopropyltriméthyl ammonium ou de diméthyldiallyl ammonium. De tels produits sont commercialisés par exemple sous les dénominations de "Celquat L 200"® et "Celquat H 100"® par la Société NATIONAL STARCH.

Lorsque le polymère cationique des compositions selon l'invention est un éther de cellulose comportant des groupements ammonium quaternaires, ce dernier est de préférence choisi parmi ceux décrits dans le brevet FR-1.492.597 et tout particulièrement les ammonium quaternaires d'hydroxyéthylcellulose ayant réagi avec un époxyde substitué par un groupement triméthyl ammonium. De tels polymères sont par exemple commercialisés sous les dénominations de "JR 400"®, "JR 125"® ou "JR 30M"® ou encore sous les dénominations de "LR 400"® ou "LR 30M"® par la Société UNION CARBIDE.

Enfin, lorsque le polymère cationique des compositions selon l'invention est un copolymère vinyl pyrrolidone/acrylate ou méthacrylate de dialkylaminoalkyle quaternisé ou non ou un acrylamide ou méthacrylamide de dialkylaminoalkyle quaternisé ou non, celui-ci est de préférence choisi parmi ceux décrits dans le brevet FR-71.03017 (2.077.143) et le brevet FR-78.17320 (2.393.573) tels que ceux commercialisés sous les dénominations de "Gafquat 734"®, "Gafquat 755"® ou sous les dénominations de "Copolymère 845, 958 et 937"® par la Société ISP. On préfère également

le copolymère vinyl pyrrolidone/ méthacrylamide de diméthylaminopropyle quaternisé tel que celui commercialisé sous la dénomination de "Gafquat HS 100"® par la Société ISP.

Selon une forme de réalisation préférée de l'invention, le polymère cationique est une cellulose cationique.

De préférence, le polymère cationique présente une masse moléculaire comprise entre environ 500 et $5.10^6$ et plus particulièrement entre 10.000 et $10^6$.

En fonction de la définition donnée ci-dessus des polymères amphotères, ceux-ci peuvent appartenir aux trois groupes suivants :

(a) le premier groupe correspond aux polymères amphotères essentiellement constitués de motifs suivants :

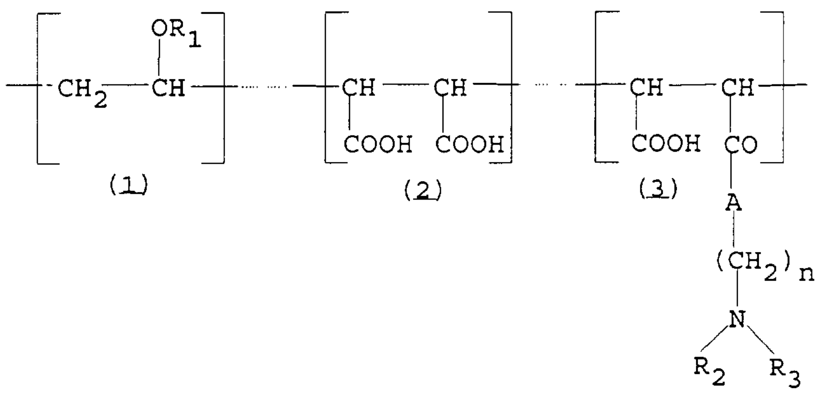

dans lesquels :

$R_1$ est un alkyle en $C_1$-$C_6$,
$R_2$ et $R_3$, identiques ou différents, représentent un alkyle en $C_1$-$C_8$
A est un atome d'oxygène ou NH, et n est 2 à 10.

De préférence, les unités de formule (1), (2) et (3) sont présentes dans les proportions en moles suivantes :

(1) de 10 à 90 % et de préférence de 20 à 50 %,
(2) de 5 à 90 % et de préférence de 15 à 50 %,
(3) de 5 à 90 % et de préférence de 15 à 50 % ;

Selon une forme particulière de réalisation, les polymères amphotères du groupe (a) sont ceux dans lesquels les radicaux $R_1$, $R_2$ et $R_3$ représentent $-CH_3$, A est NH et n = 3.

(b) le deuxième groupe correspond aux polymères amphotères essentiellement constitués de motifs suivants :

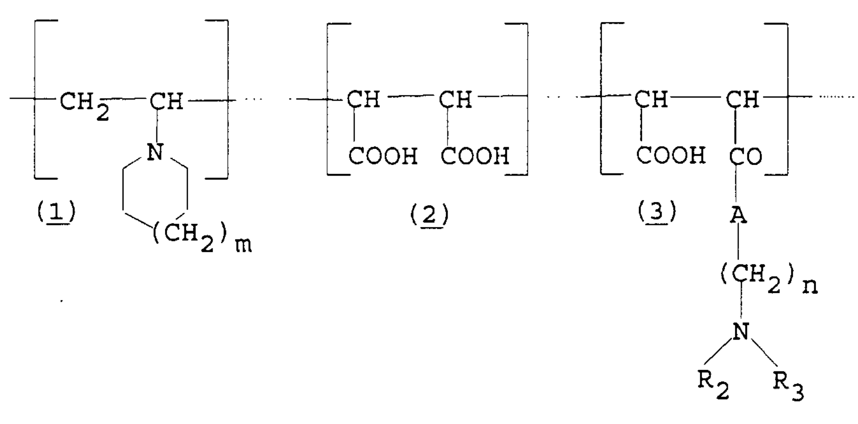

dans lesquels :

$R_2$, $R_3$, A et n ont les mêmes significations que ci-dessus, et m est 0, 1 ou 2.
De préférence, les unités de formule (1), (2) et (3) sont présentes dans les proportions en moles suivantes :

(1) de 5 à 90 % et de préférence de 10 à 70 %,
(2) de 5 à 90 % et de préférence de 10 à 70 %,
(3) de 5 à 90 % et de préférence de 10 à 70 % ;

(c) le troisième groupe correspond aux polymères amphotères essentiellement constitués de motifs suivants :

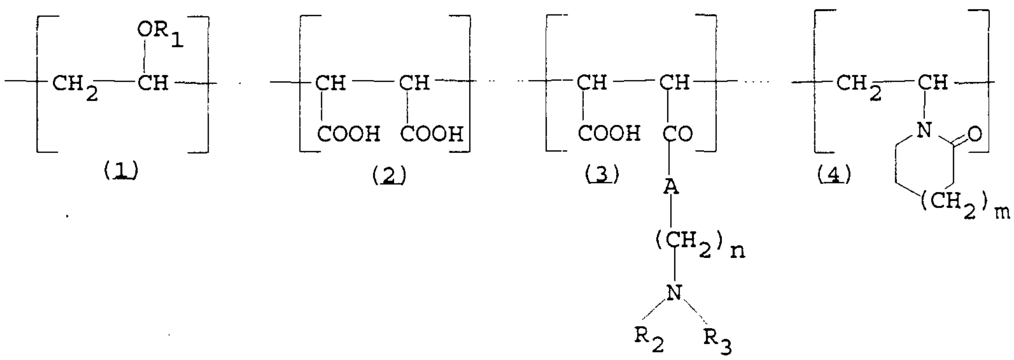

(1) (2) (3) (4)

dans lesquels :
$R_1$, $R_2$, $R_3$, A, m et n ont les mêmes significations que ci-dessus.

De préférence, les unités de formule (1), (2), (3) et (4) sont présentes dans les proportions en moles suivantes :

(1) de 5 à 90 % et de préférence de 10 à 70 %,
(2) de 5 à 90 % et de préférence de 10 à 70 %,
(3) de 5 à 90 % et de préférence de 10 à 70 %,
(4) de 5 à 90 % et de préférence de 10 à 70 %.

Selon une forme particulière de réalisation, les polymères amphotères du groupe (c) sont ceux dans lesquels les radicaux $R_1$, $R_2$ et $R_3$ représentent $-CH_3$, A est NH, n = 3 et m = 2.

Dans les polymères amphotères des groupes (a), (b) et (c) ci-dessus, les unités de formule (3) peuvent éventuellement se présenter sous forme zwittérionique.

De préférence, les polymères amphotères tels que définis ci-dessus présentent une masse moléculaire comprise entre environ 500 et $5.10^6$.

Parmi les polymères amphotères de l'invention, on peut citer par exemple ceux commercialisés par la Société ISP sous les dénominations de "ACV-4013" et "ACV-4014" et celui commercialisé par la Société ISP sous la dénomination de "Vinyl Caprolactam Modified Amphoteric Gantrez" qui sera défini ci-après sous la dénomination de "VC MAG".

Dans les compositions pour mousse capillaire selon l'invention, la proportion en polymère cationique comme en polymère amphotère peut être comprise entre environ 0,05 et 20% en poids, de préférence entre environ 0,2 et 10% en poids, et plus particulièrement entre environ 0,25 et 5% en poids, par rapport au poids total de la composition.

Le rapport en poids du polymère cationique au polymère amphotère est de préférence compris entre environ 10/90 et 90/10 et plus particulièrement entre environ 20/80 et 80/20.

Le solvant utilisé dans les compositions selon l'invention doit permettre, après expansion de la composition, la formation d'une mousse ayant de bonnes propriétés cosmétiques et qui puisse s'étaler aisément sur la chevelure.

Le solvant est de préférence constitué par de l'eau, mais peut aussi se présenter sous forme d'un mélange hydroalcoolique d'un alcool en $C_1$-$C_4$ choisi parmi l'éthanol ou l'isopropanol.

Lorsque par exemple l'on utilise une solution hydroalcoolique, la proportion en alcool n'excède pas, de préférence, 50% et est, de préférence, inférieure à 30% en poids par rapport au poids total de la composition.

Outre les polymères cationique et amphotère spécifiques décrits ci-dessus, la présente composition peut également contenir des adjuvants cosmétiques usuels qui, en eux-mêmes, n'ont pas de pouvoir moussant, tels que des colorants ayant pour fonction de colorer la composition elle-même ou les cheveux, des conservateurs, des agents

séquestrants, des agents pour régler le pH, des parfums, des silicones, des protéines, des filtres solaires, des agents traitants et/ou des électrolytes, de préférence des sels de métaux alcalins.

Le pH des compositions selon l'invention est de préférence réglé à une valeur comprise entre environ 4 et 9 et de préférence entre environ 6 et 8.

Parmi les agents régulateurs de pH, on peut notamment citer l'amino-2 méthyl-2 propanol-1, la monoéthanolamine, la triéthanol-amine, la triisopropanolamine, la soude et la potasse.

Les compositions selon l'invention peuvent être conditionnées dans des dispositifs non aérosol, notamment du type "squeeze bottle", mais on préfère tout particulièrement un conditionnement du type aérosol.

Lorsque la composition selon l'invention est conditionnée sous forme d'un aérosol, elle contient en outre un agent propulseur choisi par exemple parmi l'air comprimé, le gaz carbonique, l'azote, l'oxyde nitreux, le butane, l'isobutane, le diméthyléther, les hydrocarbures chlorés et/ou fluorés ou leurs mélanges.

La concentration en agent propulseur est généralement comprise entre 1 et 20% en poids par rapport au poids total de la composition et, de préférence, entre 5 et 15%.

La pression interne dans le récipient aérosol est généralement comprise entre environ 1 et 4 bars ($10^5$ à $4.10^5$ Pa).

Tout type de récipient et système de valve pour mousse aérosol est approprié pour la mise en oeuvre de l'invention selon cette forme de réalisation.

## TESTS DE SYNERGIE

Afin d'illustrer l'effet surprenant de synergie obtenu par l'association des polymères cationique et amphotère spécifiques décrits ci-dessus, on a réalisé les essais ci-après dont les résultats ont été regroupés dans le tableau I.

A cet effet, on a préparé des récipients pour mousses aérosol en pressurisant un mélange constitué, en proportion pondérale par rapport au poids total du mélange, de 10 % d'agent propulseur, sous la forme d'un mélange isobutane/propane/butane commercialisé sous la dénomination de "Aérogaz 3,2 N"® par la Société Elf Aquitaine, et de 90 % d'une solution aqueuse à 1 d'un polymère choisi parmi un polymère cationique, un polymère amphotère et un mélange de ces derniers.

A la sortie de l'aérosol, la mousse obtenue a été soumise à un panel de 5 juges en vue d'apprécier les critères d'expansion, de rigidité et de stabilité sur une échelle de 0 à 6, la note 0 correspondant à de faibles critères et la note 6 correspondant à d'excellents critères. Pour chaque critère examiné, on a effectué la moyenne des notes attribuées par les 5 juges.

Par ailleurs, on a mesuré la masse volumique de chaque mousse obtenue dans les conditions suivantes.

Le test est effectué 24 heures après la mise sous pression du mélange aérosol en salle conditionnée à 20°C ± 1°C, le matériel et l'échantillon étant à cette même température. Un godet cylindrique de volume V est pesé vide (soit $P_1$ son poids) puis directement rempli avec la mousse produite par l'aérosol. Chaque récipient aérosol est bien agité avant l'emploi de façon à émulsionner le gaz propulseur.

Pour une répartition uniforme de la mousse dans le godet, les aérosols sont utilisés tête en bas dans un mouvement tournant et régulier.

Dès la fin de l'expansion de la mousse, on arase immédiatement et rapidement à l'aide d'une spatule large et on pèse à nouveau le godet (soit $P_2$ son poids).

On détermine la masse volumique de la mousse selon la formule suivante :

$$\text{masse volumique à } 20°\text{C} = \frac{P_2 - P_1}{V}.$$

On effectue 3 déterminations pour chaque composition aérosol, la valeur retenue étant la valeur moyenne de ces déterminations (en $g/cm^3$).

Pour satisfaire à l'invention, la mousse doit avoir une masse volumique inférieure à 0,4 et de préférence inférieure à 0,25 $g/cm^3$.

TABLEAU I

| Polymère cationique et/ou amphotère | Concentration en polymère (% MA) * | Critères de la mousse | | | |
|---|---|---|---|---|---|
| | | Expansion (0 à 6) ** | Rigidité (0 à 6) ** | Stabilité (0 à 6) ** | Masse volumique (g/cm$^3$) |
| "Celquat L200"® (cationique) | 1 | 3,4 | 2,8 | 3,0 | 0,075 |
| "ACV-4013" (amphotère) | 1 | 1,2 | 0,7 | 0,6 | > 0,25 |
| "VC MAG" (amphotère) | 1 | 3,8 | 0,5 | 0,6 | > 0,25 |
| "Celquat L200"® (cationique) + "ACV-4013" (amphotère) | 0,5<br>0,5 | 4,7 | 4,3 | 4,9 | 0,066 |
| "Celquat L200"® (cationique) + "VC MAG" (amphotère) | 0,5<br>0,5 | 5,2 | 4,4 | 5,4 | 0,041 |

* (% MA) : % en poids de matière active
** 0 = très mauvais → 6 = très bon

Les résultats du Tableau I montrent clairement l'effet surprenant de synergie lorsque l'on associe un polymère cationique à un polymère amphotère tels que définis ci-dessus selon l'objet de la présente invention.
Les exemples suivants permettent d'illustrer l'invention.

**EXEMPLE 1** : *Mousse de coiffage*

On prépare une mousse aérosol en procédant au mélange des ingrédients suivants :

- Polymère cationique "Celquat L200"® 0,8% MA
- Polymère amphotère "VC MAG" 0,5% MA
- Amino-2 méthyl-2 propanol-1 q.s. pH = 8
- Eau déminéralisée q.s.p. 100g

On introduit la composition obtenue dans un récipient aérosol puis de l'"Aérogaz 3,2 N"® en tant qu'agent propulseur, respectivement dans des proportions de 95 % et 5 % en poids par rapport au poids total du mélange. On fixe ensuite une valve appropriée sur le récipient aérosol.
La mousse obtenue après dépressurisation est d'excellente qualité et a une masse volumique de 0,035 g/cm$^3$ selon le test tel que décrit ci-dessus.

**EXEMPLE 2** : *Mousse de coiffage*

On prépare une mousse aérosol en procédant au mélange des ingrédients suivants :

- Polymère cationique "Gafquat 734"® 1 % MA

- Polymère amphotère "VC MAG" 1 % MA
- Ethanol absolu 8,2 g
- Amino-2 méthyl-2 propanol-1 q.s. pH = 8
- Eau déminéralisée q.s.p. 100g

On introduit la composition obtenue dans un récipient aérosol puis de l'"Aérogaz 3,2 N"® en tant qu'agent propulseur, respectivement dans des proportions de 90 % et 10 % en poids par rapport au poids total du mélange et on fixe une valve appropriée sur le récipient aérosol.

La mousse obtenue après dépressurisation est d'excellente qualité et a une masse volumique de 0,03 g/$cm^3$ selon le test tel que décrit ci-dessus.

**EXEMPLE 3** : *Mousse de coiffage*

On prépare une mousse aérosol en procédant au mélange des ingrédients suivants :

- Polymère cationique "Gafquat 755"® 1 % MA
- Polymère amphotère "ACV 4013" 1,5 % MA
- Amino-2 méthyl-2 propanol-1 q.s. pH = 7
- Eau déminéralisée q.s.p. 100g

On introduit la composition obtenue dans un récipient aérosol puis de l'"Aérogaz 3,2 N"® en tant qu'agent propulseur, respectivement dans des proportions de 90 % et 10 % en poids par rapport au poids total du mélange et on fixe une valve appropriée sur le récipient aérosol. La mousse obtenue après dépressurisation est d'excellente qualité et a une masse volumique de 0,075 g/$cm^3$ selon le test tel que décrit ci-dessus.

## Revendications

1. Composition cosmétique pour mousse capillaire à base d'une association de polymères, caractérisée par le fait qu'elle contient, dans un véhicule cosmétiquement acceptable, un mélange (i) d'au moins un polymère cationique choisi parmi les celluloses cationiques, les éthers de cellulose comportant des groupements ammonium quaternaires, les copolymères de vinyl pyrrolidone et d'acrylate ou méthacrylate de dialkylaminoalkyle quaternisés ou non et les copolymères de vinyl pyrrolidone et d'acrylamide ou méthacrylamide de dialkylaminoalkyle quaternisés ou non et (ii) d'au moins un polymère amphotère choisi parmi les polymères dérivant de la copolymérisation d'un alkylvinyléther et d'anhydride maléique, d'un N-vinyl lactame et d'anhydride maléique, ou d'un alkylvinyléther, d'anhydride maléique et d'un N-vinyl lactame, lesdits polymères étant modifiés par une N,N-dialkylaminoalkylamine ou par un N,N-dialkylaminoalcanol.

2. Composition selon revendication 1, caractérisée par le fait que ledit polymère cationique est présent en une proportion comprise entre 0,05 et 20 % en poids par rapport au poids total de la composition.

3. Composition selon la revendication 1, caractérisée par le fait que ledit polymère amphotère est essentiellement constitué de motifs suivants :

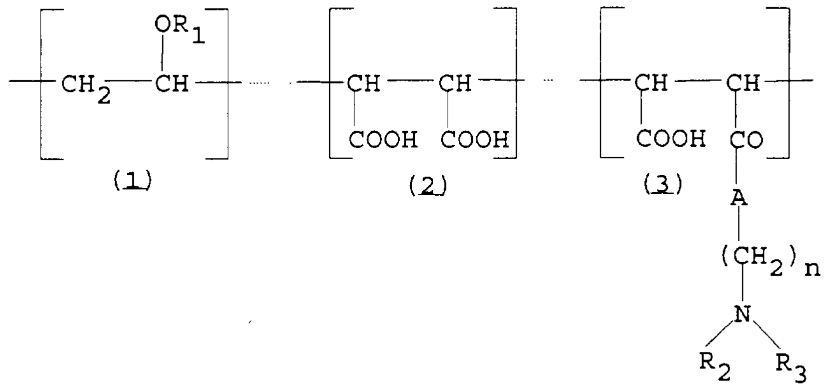

dans lesquels :

$R_1$ est un alkyle en $C_1$-$C_6$,
$R_2$ et $R_3$, identiques ou différents, représentent un alkyle en $C_1$-$C_8$
A est un atome d'oxygène ou NH, et n est 2 à 10.

**4.** Composition selon la revendication 3, caractérisée par le fait que dans les motifs du polymère amphotère $R_1$, $R_2$ et $R_3$ représentent $-CH_3$, A est NH et n = 3.

**5.** Composition selon la revendication 1, caractérisée par le fait que ledit polymère amphotère est essentiellement constitué des motifs suivants :

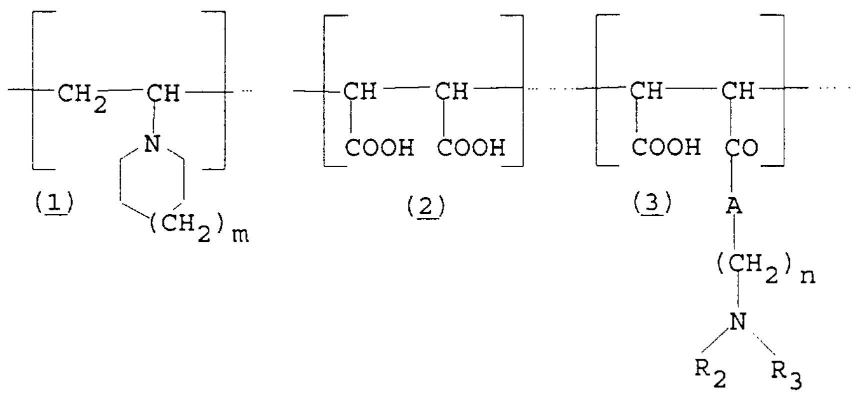

dans lesquels :
$R_2$, $R_3$ et A et n ont les mêmes significations que celles données à la revendication 3, et m est 0, 1 ou 2.

**6.** Composition selon la revendication 1, caractérisée par le fait que ledit polymère amphotère est essentiellement constitué de motifs suivants :

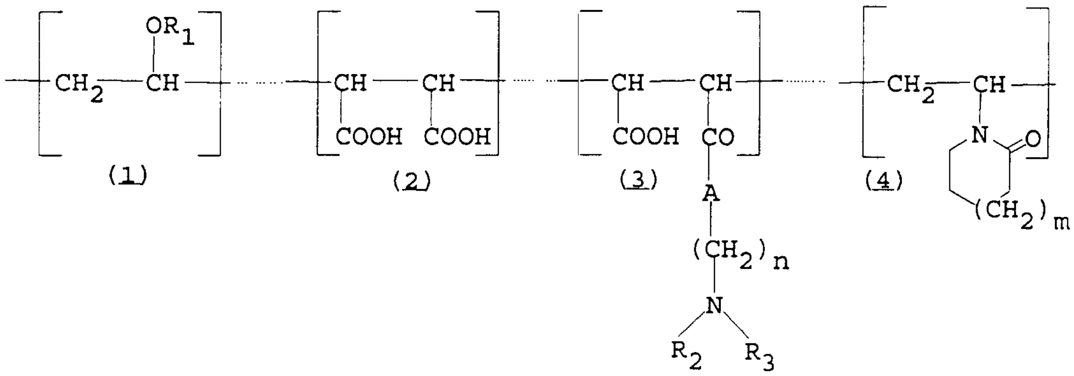

dans lesquels :

$R_1$, $R_2$, $R_3$, A et n ont les mêmes significations que celles données à la revendication 3, et
m est 0, 1 ou 2.

7. Composition selon la revendication 6, caractérisée par le fait que dans les motifs du polymère amphotère $R_1$, $R_2$ et $R_3$ représentent $-CH_3$, A est NH, n = 3 et m = 2.

8. Composition selon l'une des revendications 3 à 7, caractérisée par le fait que ledit polymère amphotère se présente sous forme zwittérionique.

9. Composition selon l'une quelconque des revendications précédentes, caractérisée par le fait que ledit polymère amphotère est présent en une proportion comprise entre 0,05 et 20 % en poids par rapport au poids total de la composition.

10. Composition selon l'une quelconque des revendications précédentes, caractérisée par le fait que le rapport en poids du polymère cationique au polymère amphotère est compris entre 10/90 et 90/10.

11. Composition selon l'une quelconque des revendications précédentes, caractérisée par le fait que ledit véhicule cosmétiquement acceptable est un solvant aqueux ou hydroalcoolique.

12. Composition selon l'une quelconque des revendications précédentes, caractérisée par le fait qu'elle contient en outre des adjuvants cosmétiques usuels choisis parmi des colorants, des conservateurs, des séquestrants, des agents régulateurs de pH, des parfums, des silicones, des protéines, des filtres solaires, des agents traitants et des électrolytes.

13. Composition selon la revendication 12, caractérisée par le fait que l'agent régulateur de pH est choisi parmi l'amino-2 méthyl-2 propanol-1, la monoéthanolamine, la triéthanolamine, la triisopropanolamine, la soude et la potasse.

14. Composition selon l'une quelconque des revendications précédentes, caractérisée par le fait que son pH est compris entre 4 et 9.

15. Composition selon l'une quelconque des revendications précédentes, caractérisée par le fait qu'elle contient en outre un agent propulseur choisi parmi l'air comprimé, le gaz carbonique, l'azote, l'oxyde nitreux, le butane, l'isobutane, le diméthyléther, les hydrocarbures chlorés et/ou fluorés, ou leurs mélanges

16. Composition selon la revendication 15, caractérisée par le fait que la mousse obtenue a une masse volumique inférieure à 0,25 g/$cm^3$.

**Patentansprüche**

1. Kosmetische Zubereitung als Haarschaum auf Basis einer Polymerassoziation, dadurch gekennzeichnet, dass

sie in einem geeigneten kosmetischen Träger eine Mischung von (i) mindestens einem kationischen Polymer, ausgewählt unter kationischen Cellulosen, Celluloseethern, die quaternäre Ammoniumgruppen tragen, Copolymeren von Vinylpyrrolidon und gegebenenfalls quaternisiertem Dialkylaminoalkylacrylat oder -methacrylat und Copolymeren von Vinylpyrrolidon und gegebenenfalls quaternisiertem Dialkylaminoalkylacrylamid oder -methacrylamid, und (ii) mindestens einem amphoteren Polymeren, ausgewählt unter den sich aus der Copolymerisation eines Alkylvinylethers und Maleinsäureanhydrid, eines N-Vinyllactams und Maleinsäureanhydrid oder eines Alkylvinylethers, Maleinsäureanhydrid und eines N-Vinyllactams ableitenden Polymeren, diese Polymeren modifiziert durch ein N,N-Dialkylaminoalkylamin oder ein N,N-Dialkylaminoalkanol, enthält.

**2.** Zubereitung gemäß Anspruch 1, dadurch gekennzeichnet, dass das kationische Polymer in einer Menge zwischen 0,05 und 20 Gew.-%, bezogen auf das Gesamtgewicht der Zubereitung, vorhanden ist.

**3.** Zubereitung gemäß Anspruch 1, dadurch gekennzeichnet, dass das amphotere Polymer im Wesentlichen aus den folgenden Einheiten aufgebaut ist:

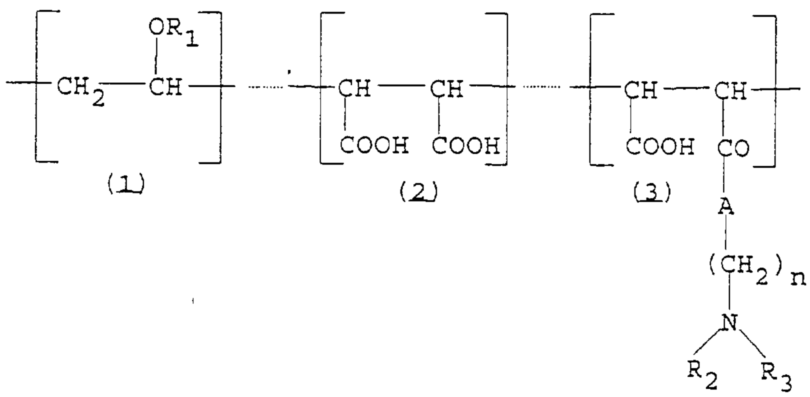

worin:

$R_1$ ein $C_1$-$C_6$-Alkyl ist,
$R_2$ und $R_3$, die gleich oder verschieden sein können, ein $C_1$-$C_8$-Alkyl darstellen, und
A ein Sauerstoffatom oder NH ist, und n 2 bis 10 bedeutet.

**4.** Zubereitung gemäß Anspruch 3, dadurch gekennzeichnet, dass in den Einheiten des amphoteren Polymers $R_1$, $R_2$ und $R_3$ -$CH_3$ bedeuten, A NH ist und n = 3 ist.

**5.** Zubereitung gemäß Anspruch 1, dadurch gekennzeichnet, dass das amphotere Polymer im Wesentlichen aus den folgenden Einheiten aufgebaut ist:

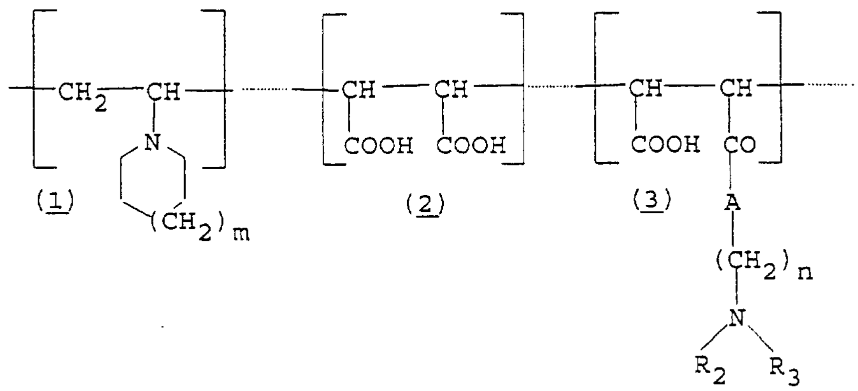

worin:
$R_2$, $R_3$ und A sowie n die in Anspruch 3 gegebene Bedeutung aufweisen und m 0, 1 oder 2 ist.

**6.** Zubereitung gemäß Anspruch 1, dadurch gekennzeichnet, dass das amphotere Polymer im Wesentlichen aus den folgenden Einheiten aufgebaut ist:

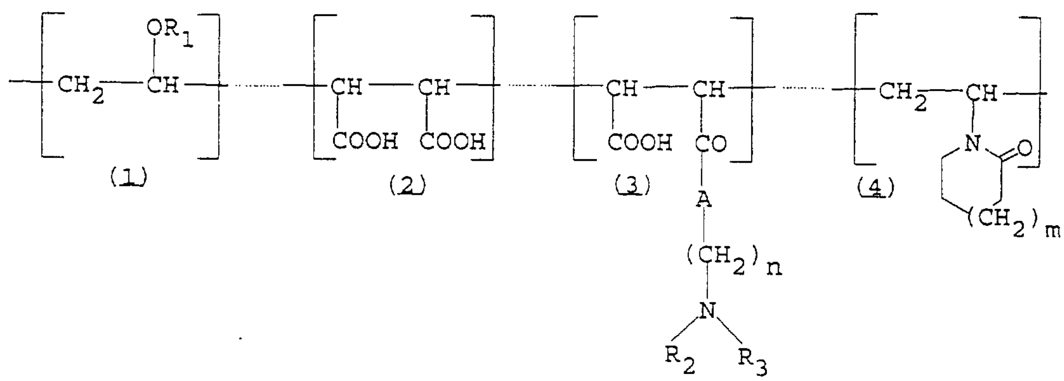

worin:

$R_1$, $R_2$, $R_3$, A und n die in Anspruch 3 angegebene Bedeutung haben und
m 0, 1 oder 2 ist.

**7.** Zubereitung gemäß Anspruch 6, dadurch gekennzeichnet, dass in den Einheiten des amphoteren Polymers $R_1$, $R_2$ und $R_3$ -$CH_3$ bedeuten, A NH ist, n = 3 und m = 2 ist.

**8.** Zubereitung gemäß einem der Ansprüche 3 bis 7, dadurch gekennzeichnet, dass das amphotere Polymer in zwitterionischer Form vorliegt.

**9.** Zubereitung gemäß einem der vorstehenden Ansprüche, dadurch gekennzeichnet, dass das amphotere Polymer in einer Menge zwischen 0,05 und 20 Gew.-%, bezogen auf das Gesamtgewicht der Zubereitung, vorhanden ist.

**10.** Zubereitung gemäß einem der vorstehenden Ansprüche, dadurch gekennzeichnet, dass das Gewichtsverhältnis von kationischem Polymer zu amphoterem Polymer von 10/90 bis 90/10 beträgt.

**11.** Zubereitung gemäß einem der vorstehenden Ansprüche, dadurch gekennzeichnet, dass es sich bei dem geeigneten kosmetischen Träger um ein wässriges oder hydroalkoholisches Lösungsmittel handelt.

**12.** Zubereitung gemäß einem der vorstehenden Ansprüche, dadurch gekennzeichnet, dass sie zusätzlich übliche kosmetische Hilfsmittel enthält, ausgewählt unter den Farbstoffen, den Konservierungsmitteln, den Sequestriermitteln, den pH-Regulatoren, den Parfümen, den Silikonen, den Proteinen, den UV-Filtern, den Behandlungsmitteln und den Elektrolyten.

**13.** Zubereitung gemäß Anspruch 12, dadurch gekennzeichnet, dass der pH-Regulator ausgewählt ist unter 2-Amino-2-methyl-1-propanol, dem Monoethanolamin, dem Triethanolamin, dem Triisopropanolamin, Soda und Pottasche.

**14.** Zubereitung gemäß einem der vorstehenden Ansprüche, dadurch gekennzeichnet, dass ihr pH zwischen 4 und 9 liegt.

**15.** Zubereitung gemäß einem der vorstehenden Ansprüche, dadurch gekennzeichnet, dass sie zusätzlich ein Treibmittel enthält, ausgewählt unter komprimierter Luft, Kohlendioxid, Stickstoff, Stickoxiden, Butan, Isobutan, Dimethylether, den chlorierten und/oder fluorierten Kohlenwasserstoffen oder Mischungen derselben.

**16.** Zubereitung gemäß Anspruch 15, dadurch gekennzeichnet, dass der erhaltene Schaum eine Dichte kleiner als 0,25 $g/cm^3$ aufweist.

## Claims

**1.** Cosmetic composition for a hair mousse based on a combination of polymers, characterized in that it contains, in a cosmetically acceptable vehicle, a mixture (i) of at least one cationic polymer chosen from cationic celluloses, cellulose ethers containing quaternary ammonium groups, copolymers of vinylpyrrolidone and of dialkylaminoalkyl acrylate or methacrylate, which may or may not be quaternized, and copolymers of vinylpyrrolidone and of dialkylaminoalkylacrylamide or dialkylaminoalkylmethacrylamide, which may or may not be quaternized, and (ii) of at least one amphoteric polymer chosen from polymers derived from the copolymerization of an alkyl vinyl ether and of maleic anhydride, of an N-vinyllactam and of maleic anhydride, or of an alkyl vinyl ether, maleic anhydride and an N-vinyllactam, the said polymers being modified with an N,N-dialkylaminoalkylamine or with an N,N-dialkylaminoalkanol.

**2.** Composition according to Claim 1, characterized in that the said cationic polymer is present in a proportion of between 0.05 and 20% by weight relative to the total weight of the composition.

**3.** Composition according to Claim 1, characterized in that the said amphoteric polymer consists essentially of the following units:

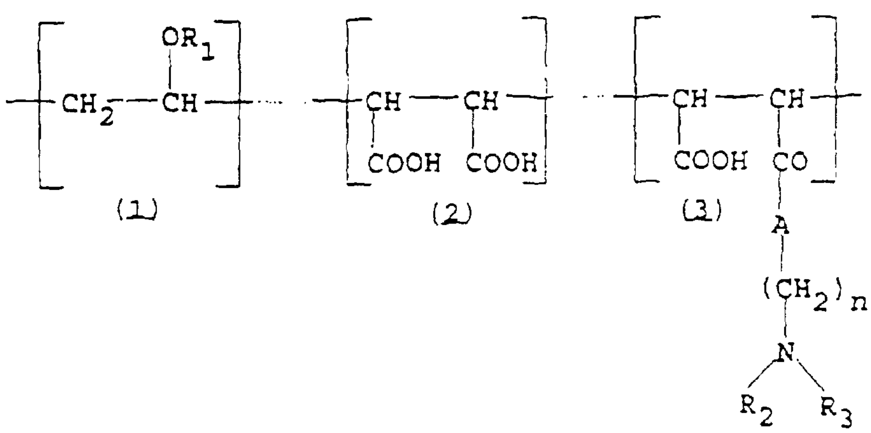

in which:

$R_1$ is a $C_1$-$C_6$ alkyl,
$R_2$ and $R_3$, which may be identical or different, represent a $C_1$-$C_8$ alkyl,
A is an oxygen atom or NH, and n is 2 to 10.

4. Composition according to Claim 3, characterized in that, in the units of the amphoteric polymer, $R_1$, $R_2$ and $R_3$ represent $-CH_3$, A is NH and n = 3.

5. Composition according to Claim 1, characterized in that the said amphoteric polymer consists essentially of the following units:

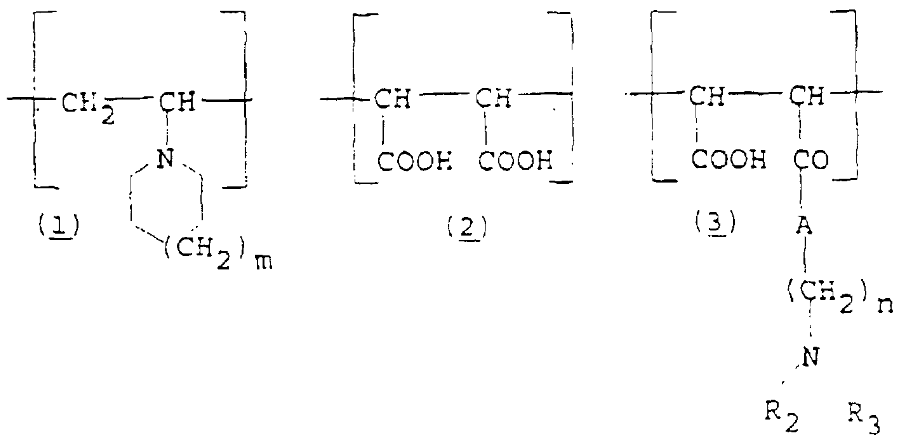

in which:

$R_2$, $R_3$, A and n have the same meanings as those given in Claim 3, and m is 0, 1 or 2.

6. Composition according to Claim 1, characterized in that the said amphoteric polymer consists essentially of the following units:

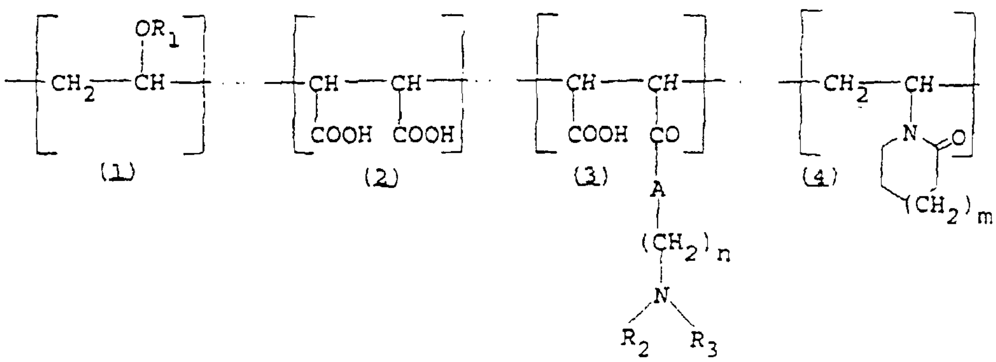

in which:

$R_1$, $R_2$, $R_3$, A and n have the same meanings as those given in Claim 3, and m is 0, 1 or 2.

7. Composition according to Claim 6, characterized in that, in the units of the amphoteric polymer, $R_1$, $R_2$ and $R_3$ represent $-CH_3$, A is NH, n = 3 and m = 2.

8. Composition according to one of Claims 3 to 7, characterized in that the said amphoteric polymer is in zwitterionic form.

9. Composition according to any one of the preceding claims, characterized in that the said amphoteric polymer is present in a proportion of between 0.05% and 20% by weight relative to the total weight of the composition.

10. Composition according to any one of the preceding claims, characterized in that the weight ratio of the cationic

polymer to the amphoteric polymer is between 10/90 and 90/10.

**11.** Composition according to any one of the preceding claims, characterized in that the said cosmetically acceptable vehicle is an aqueous or aqueous-alcoholic solvent.

**12.** Composition according to any one of the preceding claims, characterized in that it also contains common cosmetic adjuvants chosen from colorants, preserving agents, sequestering agents, pH regulators, fragrances, silicones, proteins, sunscreens, treating agents and electrolytes.

**13.** Composition according to Claim 12, characterized in that the pH regulator is chosen from 2-amino-2-methyl-1-propanol, monoethanolamine, triethanolamine, triisopropanolamine, sodium hydroxide and potassium hydroxide.

**14.** Composition according to any one of the preceding claims, characterized in that its pH is between 4 and 9.

**15.** Composition according to any one of the preceding claims, characterized in that it also contains a propellant chosen from compressed air, carbon dioxide, nitrogen, nitrous oxide, butane, isobutane, dimethyl ether, chlorohydrocarbons and/or fluorohydrocarbons, or mixtures thereof.

**16.** Composition according to Claim 15, characterized in that the mousse obtained has a specific mass of less than 0.25 $g/cm^3$.